# EUROPÄISCHE PATENTSCHRIFT

(11) **EP 0 591 624 B1**
(45) Veröffentlichungstag und Bekanntmachung des Hinweises auf die Patenterteilung: **05.06.1996**
(21) Anmeldenummer: 93110631.4
(22) Anmeldetag: 03.07.1993
(51) Int. Cl.: C07D 213/61, C07D 213/64

(54) **Verfahren zur Herstellung von reinem 2,5-Dichlorpyridin sowie Gewinnung des als Nebenprodukt anfallenden 2,3-Dichlorpyrdins**
Process for the preparation of pure 2,5-dichloropyridine and the isolation of 2,3-dichloropyridine which is formed as a by-product
Procédé pour la préparation de 2,5-dichloropyridine pure et l'isolation de 2,3-dichloropyridine qui est obtenu comme sous-produit

(30) Priorität: 07.10.1992 DE 4233708
(43) Veröffentlichungstag der Anmeldung: 13.04.1994
(73) Patentinhaber: RÜTGERSWERKE AKTIENGESELLSCHAFT, 60326 Frankfurt (DE)
(72) Erfinder: Sendelbach, Stefan, Dr., D-7107 Neckarsulm (DE); Orth, Winfried, Dr., D-6733 Hassloch/Pfalz (DE); Weiss, Wolfgang, Dr., D-6803 Edingen-Neckarhausen (DE); Kleffner, Hans Werner, Dr., D-6719 Battenberg/Plafz (DE); Läufer, Albrecht, Dr., D-6940 Weinheim (DE)

(56) Entgegenhaltungen:
- JOURNAL OF THE CHEMICAL SOCIETY, PERKIN TRANSACTIONS 2 1966 , LETCHWORTH GB Seiten 991 - 995 E. SPINNER; J.C.B. WHITE 'Spectral and ionisation constant studies of substituted 2-hydroxypyridines'
- SYNTHETIC COMMUNICATIONS Bd. 20, Nr. 19 , 1990 Seiten 2971 - 2977 MIN-JEN SHIAO ET AL. 'A convenient synthesis of halogenated 2-chloropyridines...'
- JOURNAL OF THE CHEMICAL SOCIETY, PERKIN TRANSACTIONS 2 1968 , LETCHWORTH GB Seiten 492 - 496 R.A. ABRAMOVITCH ET AL. 'Aromatic Substitution. Part XVIII.'
- CHEMICAL ABSTRACTS, vol. 111, no. 15, 1989, Columbus, Ohio, US; abstract no. 134004y, M. UMENO ET AL. 'Preparation of 2,3,5-trichloropyridine from 2-chloropyridine via 2-hydroxypyridine' Seite 746 ;

## Beschreibung

Die Erfindung betrifft ein neues Verfahren zur Herstellung von 2,5-Dichlorpyridin als isomerenfreies Produkt aus 2-Chlorpyridin oder 2-Brompyridin. Das neue Verfahren schließt auch die Gewinnung und Reindarstellung der in 3-Stellung chlorierten Nebenprodukten ein.

Sowohl das 2,5- als auch das 2,3-Dichlorpyridin stellen wertvolle Zwischenprodukte für die Herstellung von Agrochemikalien, wie z. B. Herbizide oder Insektizide dar. Sie dienen aber auch zur Synthese von Pharmazeutika, wie z. B. von Verbindungen mit antidepressiver oder spannungslösender Wirkung.

Das bislang in größeren Mengen verbrauchte 2,5-Dichlorpyridin wird üblicherweise nach einem in DE-PS 1 695 659 beschriebenen Verfahren aus 2-Aminopyridin hergestellt. Dieses Verfahren beruht auf einer Dediazotierungsreaktion, wobei die Aminogruppe, die ursprünglich dazu gedient hat, die Chlorierung in para-Stellung zu dirigieren, durch Chlor als Substituenten ersetzt wird. Diese Substitution erfolgt in Gegenwart von Kupfer oder Kupferchlorid. Das als Nebenprodukt anfallende 5-Chlorpyridin-2-on wird durch Behandeln mit POCl₃ in DMF ebenfalls in das gewünschte 2,5-Dichlorpyridin überführt. Gravierender Nachteil dieses Verfahrens ist nicht nur die geringe Ausbeute der ersten Chlorierungsstufe von etwa 63 % der Theorie sondern sind auch die toxischen Eigenschaften des 5-Chlor-2-aminopyridins, das auch in geringsten Spuren in pharmazeutischen Produkten unerwünscht ist, obwohl es toxikologisch als mindergiftig eingestuft ist.

Neben diesem im vergleichsweise größeren Umfang durchgeführten Verfahren sind noch weitere Methoden zur Herstellung von 2,5-Dichlorpyridin bekannt, die jedoch alle mit erheblichen Nachteilen behaftet sind.

So ist aus US 3 947 457 ein Verfahren bekannt, durch das 2,5-Dichlorpyridin aus 2,3,4,5-Tetrachlorpyridin in 91 %iger Ausbeute durch Behandlung mit Hydrazin in Ethanol in Gegenwart von Triethylenamin erhalten wird. Aufgrund der Kanzerogenität von Hydrazin läßt sich dieses Verfahren jedoch nicht in technischem Maßstab durchführen.

Weiterhin wird durch zwei japanische Schriften (JP 01/121 267 A2, JP 58/206 564 A2) die Chlorierung von 2-Chlorpyridin mit Cl₂ in Gegenwart von Eisen-III-chlorid-Katalysatoren beschrieben. Nachteilig ist jedoch, daß zur Durchführung dieser Chlorierungsmethoden hohe Temperaturen benötigt werden und daß die Reaktion eine verhältnismäßig geringe Selektivität besitzt. Es entstehen in erheblichen Mengen andere Di-, Tri- und Tetrachloride.

Aufgabe der Erfindung ist es daher, ein wirtschaftliches Verfahren zur Verfügung zu stellen, durch das 2,5-Dichlorpyridin in hohen Ausbeuten rein hergestellt werden kann, was aber weder zur Bildung von unerwünschten toxischen Nebenprodukten führt, noch mit dem Einsatz kanzerogener Substanzen verbunden ist.

Die Lösung der Aufgabe erfolgt durch ein Verfahren gemäß Anspruch 1 und seiner wirtschaftlicheren Variation gemäß Anspruch 2, wobei als wertvolles Nebenprodukt anfallendes 2-Alkoxy-3-chlorpyridin gemäß Anspruch 3 abgetrennt und zu 2,3-Dichlorpyridin umgesetzt wird. Insbesondere erfolgt die Lösung der Aufgabe durch die besondere Ausgestaltung des Verfahrens gemäß der Ansprüche 4 bis 13. Als neues Zwischenprodukt wird 2-Butoxy-5-Chlorpyridin gemäß Anspruch 14 gewonnen.

Aus Chem. Techn. 10 (1958), 145 und JP 64/75468 sind Verfahren bekannt, durch die 2-Chlorpyridin in 2-Stellung durch einen Alkohol alkoxyliert werden kann. Durch eine anschließende Chlorierung werden Gemische von Chlorierungsprodukten erhalten, in denen unter anderem auch 5-Chlor-2-alkoxypyridine erhalten sind. (Synth. Comm. 20 (19), 2971-2977 (1990); J. Chem. Soc. (B), (1966) 991-995); Synth. Comm. 19, 1505 (1989). Je nach weiterer Aufarbeitung können daraus 2-Chlorpyridine (Synth. Comm. 20, 2971 (1990) oder 2-Pyridone (J. Org. Chem. 23 1287 (1958) gewonnen werden.

Es wurde nun gefunden, daß die Chlorierung des 2-Alkoxypyridins zugunsten des in 5-Stellung chlorierten Produktes verschoben werden kann, wenn die Reaktion bei Raumtemperatur in einem wäßrigen Medium in Gegenwart einer Hilfsbase und eines Katalysators chloriert wird. Die Reaktion kann beschleunigt werden, wenn dem Reaktionsmedium geringe Mengen eines Emulgators zugefügt werden. Besonders hohe Ausbeuten an 5-Chlor-2-alkoxypyridin werden erzielt, wenn es sich bei der Alkoxygruppe um einen Rest mit 4 bis 8 C-Atomen handelt. Beispielsweise werden durch eine entsprechende Chlorierung von 2-Butoxypyridin zu 85 % 5-Chlor-2-butoxypyridin, etwa 14 % 3-Chlor-2-butoxypyridin und weniger als 1 % 3,5-Dichlor-2-butoxypyridin bei einer Gesamtausbeute von 90 % der Theorie erhalten.

5-Chlor-2-butoxypyridin ist ein Zwischenprodukt für die Herstellung des Pharmarohstoffes 2-Hydroxy-5-chlorpyridin durch hydrolytische Spaltung mit Hilfe von Säuren, wie z.B. HCl und bietet gegenüber den herkömmlicherweise eingesetzten Methoxy- oder Ethoxyderivaten einen herovrragenden Vorteil: Das bei der Hydrolyse mit HCl entstehende Butylchlorid ist auch unter Reaktionsbedingungen nicht flüchtig. Eine aufwendige Abluftaufbereitung kann somit unterbleiben. Zudem ist auch das destillativ leicht abtrennbare Butylchlorid ein hochwertiges, technisch verwertbares Zwischenprodukt.

Dieses hat den Vorteil, daß die beiden Hauptprodukte gemeinsam unter den Bedingungen einer Vilsmeyer-Haack-Reaktion einer chlorierenden Etherspaltung unterzogen werden können, wobei als Reaktionsprodukte 2,5-Dichlorpyridin, 2,3-Dichlorpyridin und der entsprechende Chlorkohlenwasserstoff, wie z. B. n-Butylchlorid entstehen. Dieses bedeutet, daß durch die Verwendung von Alkoholen mit 4 und mehr C-Atomen nicht nur die Ausbeute der erwünschten Chlorierungsprodukte erhöht, sondern auch noch nach einem umweltfreundlicheren Verfahren gearbeitet werden kann, da die entstehenden Chlorkohlenwasserstoffe bei der Reaktionstemperatur nicht flüchtig sind.

Im weiteren Verlauf des Verfahrens läßt sich das erhaltene Dichlorpyridin-Isomerengemisch in einfacher Weise durch Wasserdampfdestillation aufarbeiten, wobei sich ein Feststoff mit einem Gehalt von bis zu 96 % 2,5-Dichlorpyridin bildet und ein Öl abscheidet, in dem das 2,3-Dichlorpyridin auf bis zu 40 % angereichert ist. Durch einfache Umkristallisation aus einem Alkohol/Wasser-Gemisch läßt sich das 2,5-Dichlorpyridin in bis zu 100 %iger Reinheit gewinnen. 2,3-Dichlorpyridin, das u. a. noch mit dem entstandenen chlorierten Kohlenwasserstoff verunreinigt ist, läßt sich in einfacher Weise in an sich bekannter Weise rein gewinnen.

Eine besondere Ausgestaltung des Verfahrens besteht darin, daß das 2-Alkoxy-Chlorpyridin-Isomerengemisch in einem Lösungsmittel aufgenommen wird und das 5-Isomere durch Einleiten eines Halogenwasserstoffes als Ammoniumsalz ausgefällt wird, wobei das 3-Isomere in Lösung bleibt. Nach der Neutralisation mit einer Lauge wird auf diese Weise isomerenreines 5-Chlor-2-alkoxypyridin gewonnen. Beide Isomere, 5-Chlor-2-alkoxypyridin und das isolierte 3-Chlor-2-alkoxypyridin lassen sich nun getrennt in oben beschriebener Weise zu den Dichlorpyridinen umsetzen.

Zur Durchführung des Verfahrens wird der zur Alkoxylierung verwendete Alkohol in der 2,5 bis 4-fachen molaren Menge, bezogen auf das zu alkoxylierende 2-Chlorpyridin, in einem Reaktionsgefäß vorgelegt und unter Kühlung mit der 1,2- bis 1,7-fachen molaren Menge Base, ebenfalls bezogen auf 2-Chlorpyridin, versetzt, wobei die Temperatur möglichst bei 25 bis 40 °C gehalten wird. Die Alkoxylierungsreaktion findet unter Stickstoffatomsphäre statt und zwar bevorzugt bei einer Temperatur von etwa 110 °C. Es ist aber auch möglich, unter Rückflußtemperatur zu arbeiten. Die Aufarbeitung der Alkoxylierungsprodukte erfolgt in üblicher Weise durch Abtrennung des gebildeten Alkali- bzw. Erdalkalichlorids und Abdestillation des überschüssigen Alkohols.

Als Alkoxylierungsmittel können alle bei Raumtemperatur flüssigen Alkohole mit 4 bis 10 C-Atomen verwendet werden. Bevorzugt werden solche mit 4 bis 8 C-Atomen verwendet. Insbesondere sind dieses n-, i- und t-Butanol, alle verzweigten und geradkettigen primären, sekundären und tertiären Pentanole, Hexanole, Heptanole und Oktanole, soweit deren Alkoholate und entsprechenden Chloride flüssig sind, da sonst die Reaktionstemperatur erhöht werden müßte und dieses die Ausbeute des Alkoxylierungsprodukts erniedrigen würde. Zur Herstellung der Alkoholate sind alle Alkali- und Erdalkalihydroxide geeignet, insbesondere jedoch NaOH, LiOH und KOH.

Zur Chlorierung des erhaltenen 2-Alkoxypyridins wird letzteres mit der 10- bis 20-fachen, bevorzugt 15- bis 19-fachen molaren Menge destillierten Wassers, bis zu einem Prozent Emulgator, bis zu einem Prozent Katalysator, sowie etwa einem 10tel der nach und nach zuzudosierenden Gesamtmenge der Hilfsbase, etwa 0,5 bis 0,8 Mol pro Mol 2-Alkoxypyridin, bei Raumtemperatur in einen Reaktionsgefäß vorgelegt. Unter Rühren und beibehaltener Temperatur wird solange Chlorgas eingeleitet bis die Reaktion beendet ist. Während der Reaktion wird die Hilfsbase portionsweise nachdosiert, wenn der pH-Wert unter 7 sinkt und die vorher zudosierte Hilfsbase völlig gelöst ist.

Die Zufuhr des Chlorgases wird möglichst so geführt, daß bis zum Reaktionsende etwa die 1,1 bis 1,5-fache molare Menge, bezogen auf das 2-Alkoxypyridin, verbraucht werden.

Als Emulgator können in dieser Reaktion solche verwendet werden, die in Gegenwart von reaktivem Chlor ihre Reaktivität beibehalten, insbesondere Alkylsulfonate, und zwar solche mit 10 bis 18 C-Atomen. Von besonderem Vorteil ist, daß der Emulgator bei Raumtemperatur niedrig dosiert werden kann. So können bereits 0,1 %, bezogen auf die Gesamtmenge, ausreichen. Im allgemeinen werden bis zu 1 % Emulgator verwendet.

Als Hilfsbase kann während der Reaktion jedes Alkali- oder Erdalkalioxyd oder -hydroxid wirken, wie Li₂O, MgO, CaO, BaO, NaOH, KOH, LiOH, Mg(OH)₂, Ca(OH)₂, Ba(OH)₂, Na₂CO₃, K₂CO₃, Li₂CO₃, MgCO₃, CaCO₃, BaCO₃ oder Alkali- oder Erdalkaliacetat. Bevorzugt werden die Erdalkalioxyde verwendet. Besonders bevorzugt wird MgO für diesen Zweck eingesetzt.

Bei dem während der Reaktion eingesetzten Katalysator, kann es sich um Jod oder Brom handeln, das wie auch der Emulgator sehr gering dosiert werden kann. Es reichen bereits etwa 0,1 bis 1 %, bezogen auf die Gesamtreaktionsmenge aus, um eine ausreichende Wirksamkeit zu erzielen. In manchen Fällen ist es sogar möglich ganz auf die Zugabe eines Katalysators zu verzichten.

Nachdem die Chlorierung abgeschlossen ist, wird die Reaktionslösung durch Zugabe eines Carbonats auf etwa pH 5 bis 6 eingestellt und die sich bildenden Phasen getrennt aufgearbeitet, und zwar durch Destillation bei 13.3 millibar (10 Torr) und Kp = 75 °C, wobei das Isomerengemisch zwar nicht aufgetrennt werden kann aber von Nebenprodukten, Emulgatoren, Katalysatoren und Chlorsalzen gereinigt wird. Das erhaltene Isomerengemisch, bestehend aus bis zu 85 % 5-Isomerem und ungefähr 15 % 3-Isomerem wird anschließend in bekannter Weise, wie z. B. aus Synthetic Communications 20 (19, 2971 bis 2977 (1990) bekannt ist, durch Behandlung mit einem Vilsmeyer-Haack-Reagens in 2-Stellung chloriert. Das durch diese Reaktion erhaltene Dichlorpyridinisomerengemisch wird einer Wasserdampfdestillation unterzogen, wodurch sich eine feste Phase und ein Öl bilden.

In dem Feststoff werden 85 bis 96 % 2,5-Dichlorpyridin und geringe Mengen 2,3-Dichlorpyridin gefunden. Daraus läßt sich das 2,5-Isomere durch Umkristallisation aus einen Alkohol/Wasser-Gemisch in bis zu 100 %iger Reinheit gewinnen.

Als Alkohol kann zu diesem Zweck ein Alkohol aus der Gruppe sek. oder t-Isopropanol, n-, i-, t-Butanol, ein Pentanol oder Hexanol, ausgenommen Cyclohexanol verwendet werden.

Das im abgetrennten Öl enthaltene 2,3-Dichlorpyridin läßt sich nach in der Literatur beschriebenen Methoden rein gewinnen.

### BEISPIELE

### Beispiel 1 2-Butoxypyridin aus 2-Chlorpyridin

Zu einer auf 100 °C erwärmten Lösung von 55 g (1,38 mol) Natriumhydroxid in 245 g n-Butanol tropft man innerhalb von vier Stunden 113,5 g (1 mol) 2-Chlorpyridin. Man läßt etwa 16 Stunden bei dieser Temperatur nachrühren und erhitzt anschließend 2 Stunden zum Rückfluß (ca. 112 °C). Nach dem Erkalten wird ausgefallenes Natriumchlorid abfiltriert und der Rückstand mit ca. 500 ml n-Butanol gewaschen. Die vereinigten Filtrate werden im Vakuum destilliert. Zunächst destilliert ein Butanol-Wasser Azeotrp über, dann folgen n-Butanol und ein 2-Chlorpyridin/n-Butanol-Zwischenlauf. Das 2-Butoxypyridin destlliert zuletzt bei 75 °C/13.3 millibar (10 Torr) als farblose Flüssigkeit über.

Ausbeute 108 g (72 %) 2-Butoxypyridin mit einer Reinheit von > 98 %.

### Beispiels 2 Chlorierung

1 000 g (6,6 mol) 2-Butoxypyridin, 2 207 g (122 mol) destilliertes Wasser, 1 g Alkylsulfonat (mit 13 bis 18 C-Atomen/Molekül, z. B. Hostapur SAS 60), 6,6 g (0,03 mol) Jod sowie etwa 17 g MgO als Base werden in einen mit Rührer versehenen 6 l-Kolben vorgelegt und bei Raumtemperatur (23 bis 25 °C) gerührt. Es wird nun solange Chlorgas eingeleitet, bis das hinzugefügte MgO völlig aufgelöst ist und der pH-Wert < 7 ist. Dieser Vorgang wird so häufig wiederholt, bis insgesamt 176 g (4,4 mol) MgO verbraucht worden sind. Da die Reaktion exotherm verläuft, muß die Temperatur während der gesamten Reaktionszeit durch Kühlen bei unter 30 °C gehalten werden. Die gesamte Chlormenge beträgt 606 g (8,5 mol). Nach Beendigung der Reaktion wird noch 30 min. nachgerührt und weiterhin gekühlt, wodurch überschüssiges Chlorgas entfernt wird und Nachreaktionen, die die Temperaturen steigen ließen, vermindert werden. Der pH-Wert des Reaktionsgemischs beträgt nun etwa 1. Durch portionsweise Zugabe von Natriumhydrogencarbonat wird der pH-Wert auf etwa 5 bis 6 eingestellt.

In einem 6 l Ausrührkolben erfolgt innerhalb von etwa 10 min eine Phasentrennung in eine wäßrige hellgelbe und eine organische intensiv gelbe Phase. Die Phasen werden getrennt und die organische Phase im Vakuum destilliert. Ausbeute 1 110 g (90 %) Kp₁₄ 117 bis 119 °C. Das Produkt besteht nach GC und 1H-NMR aus 85 % 5-Chlor-2-butoxypyridin un 15 % 3-Chlor-2-butoxypyridin.

Chloreinleitzeit von 7 Stunden, eingeleitete Chlormenge: 606 g (8,5 mol)
Ausbeute: 90 %

### Beispiel 3 Herstellung von 2,3- und 2,5-Dichlorpyridin

Zu 231 g (1,5 mol) Phosphoroxichlorid tropft man bei 100 °C innerhalb 5 Stunden eine Lösung von 112 g (0,6 mol) Chlorbutoxypyridin-Gemisch [aus Beispiel 2] in 105 g (1,5 mol) Dimethylformamid. Danach läßt man 20 Stunden bei 100 °C nachrühren. Anschließend destilliert man im Vakuum n-Butylchlorid und Phosphoroxichlorid ab. Der erkaltete flüssige Rückstand wird unter Kühlung mit 500 ml Wasser hydrolysiert und mit 270 g 50 %iger Natronlauge neutralisiert. Durch Wasserdampfdestillation wird das 2,5-Dichlorpyridin sowie 2,3-Dichlorpyridin und wenig n-Butylchlord gereinigt. Der aus dem Wasser abgeschiedene Feststoff wird abgesaugt.

Ausbeute 62 g (70 %). Gemisch an 2,5- und 2,3-Dichlorpyridin.

### Beispiel 4 2,5-Dichlorpyridin

62 g Gemisch aus Beispiel 3 werden aus 200 g Isopropanol/Wasser-Gemisch (15 : 85) umkristallisiert.

Ausbeute 42 g 2,5-Dichlorpyridin (62 %, bezogen auf den ursprünglichen Anteil an 2,5-Dichlorpyridin im Ausgangsmaterial (85 %) 80 % Ausbeute. Schmelzpunkt 58 bis 59 °C (Lit¹ 59 bis 60 °C)
Lit¹ = H. B. den Hertog et al. Recueil trav. chim. 69 (1950), 673

### Beispiel 5 Isomerentrennung von 5-Chlor-2-butoxypyridin und 3-Chlor-2-butoxypyridin

In eine Lösung von 1 Mol des Chlorbutoxypyridin-Gemischs (85 % 5-, 15 % 3-Isomer) in Toluol leitet man unter Kühlung 0,85 Mol trockenes Chlorwasserstoffgas ein. Man filtriert das ausgefallene 5-Chlor-2-butoxypyridin-Hydrochlorid ab (¹H-NMR [CDCl₃] : = 1,00 (t, 3H, CH₃), 1,55 (sext, 2H, Butyl-3-CH₂), 1,95 (quint., 2H, Butyl-2-CH₂), 4,62 (t, 2H, O-CH₂), 7,35 (d, 1H, Pyridyl-3H), 8,20 (dd, 1H, Pyridyl-4H), 8,38 (d, 1H, Pyridyl-6-H), 9,20 (br s, 1H, NH).

Das Hydrochlorid wird mit Natronlauge neutralisiert und das 5-Chlor-2-butoxypyridin abgetrennt. Aubeute 133 g (bezogen auf den Gesamtgehalt) entspricht 85 % der Theorie.

## Patentansprüche

1. Verfahren zur Herstellung von 2,5-Dichlorpyridin, **dadurch gekennzeichnet**, daß
a) 2-Chlorpyridin oder 2-Brompyridin in Gegenwart einer Base, insbesondere eines Alkali- oder Erdalkalihydroxyds, bei erhöhter Temperatur, und zwar bis zur Rückflußtemperatur durch einen bei Raumtemperatur flüssigen Alkohol mit 4 bis 10 C-Atomen alkoxyliert,
b) das Alkoxylierungsprodukt bei Raumtemperatur in wäßriger Suspension in Gegenwart eines Alkali- oder Erdalkalihydroxyds oder -oxyds als Hilfsbase, eines Katalysators in geringen Mengen und evtl. geringe Mengen Emulgator durch Einleiten von Chlorgas chloriert,
c) das entstandene Isomerengemisch mit einem Vilsmeyer-Haack-Reagens behandelt wird,
d) das so erhaltene 2,5- und 2,3-Isomerengemisch einer Wasserdampfdestillation unterzieht, und
e) das kristalline Produkt aus einem Alkohol/Wasser-Gemisch umkristallisiert wird.

2. Verfahren zur Herstellung von reinem 2,5-Dichlorpyridin gemäß Anspruch 1, **dadurch gekennzeichnet**, daß das entstandene und destillierte Isomerengemisch in einem aromatischen Lösungsmittel aufgenommen wird und 2-Alkoxy-5-Cl-Pyridin durch Einleiten von gasförmigem Halogenwasserstoff und Ausfällen des entstehenden Hydrochlorids vom 3-Isomeren abgetrennt wird, das 5-Isomere mit einem Vilsmeyer-Haack-Reagens behandelt wird, das erhaltene rohe 2,5-Dichlorpyridin einer Wasserdampfdestillation unterzogen wird und das kristalline Produkt aus einem Alkohol/Wasser-Gemisch umkristallisiert wird.

3. Verfahren gemäß Anspruch 2, **dadurch gekennzeichnet**, daß das durch Einleiten von gasförmigem Halogenwasserstoff in Lösung verbliebene, vom 2-Alkoxy-5-Cl-Pyridin abgetrennte, 2-Alkoxy-3-Cl-Pyridin
a) durch Destillation vom Lösungsmittel befreit wird,
b) mit einem Vilsmeyer-Haack-Reagens behandelt,
c) einer Wasserdampfdestillation unterzogen und
d) das mit 2,3-Dichlorpyridin angereicherte Öl in bekannter Weise aufgearbeitet wird.

4. Verfahren nach Anspruch 1 oder 2, **dadurch gekennzeichnet**, daß 2-Chlorpyridin oder 2-Brompyridin in Gegenwart einer Base, insbesondere eines Alkali- oder Erdalkalihydroxyds bei einer Temperatur von < 110 °C alkoxyliert wird.

5. Verfahren gemäß Anspruch 1 oder 2, **dadurch gekennzeichnet**, daß als Base oder Hilfsbase Li₂O, MgO, CaO, BaO, NaOH, KOH, LiOH, Mg(OH)₂, Ca(OH)₂, Ba(OH)₂, Na₂CO₃, K₂CO₃, Li₂CO₃, MgCO₃, CaCO₃, BaCO₃ oder ein Alkali- oder Erdalkaliacetat verwendet wird.

6. Verfahren gemäß Anspruch 1 oder 2, **dadurch gekennzeichnet**, daß als Katalysator Jod oder Brom in einer Menge von weniger als 1 %, bezogen auf die molare Menge des eingesetzten Alkoxypyridins, dient.

7. Verfahren gemäß Anspruch 1 oder 2, **dadurch gekennzeichnet**, daß ein in Gegenwart von Chlor reaktiv bleibender Emulgator in einer Menge von weniger als 1 %, bezogen auf die Menge des eingesetzten Alkoxypyridins, dient.

8. Verfahren gemäß Anspruch 1 oder 2, **dadurch gekennzeichnet**, daß als Emulgator ein Alkylsulfonat mit 10 bis 18 C-Atomen in einer Menge von weniger als 1 %, bezogen auf die Menge des eingesetzten Alkoxypyridins dient.

9. Verfahren gemäß Anspruch 1 oder 2, **dadurch gekennzeichnet**, daß die chlorierende Etherspaltung durchgeführt wird, indem ein Teil 2-Alkoxy-chlorpyridin mit 2,5 bis 3,5 Teilen POCl₃ in 2 bis 4 Teilen DMF, bezogen auf die molare Menge, zur Reaktion vermischt werden.

10. Verfahren gemäß Anspruch 1 oder 2, **dadurch gekennzeichnet**, daß zur Umkristallisation ein Gemisch aus Wasser und einem Alkohol aus der Gruppe sek. oder tert. Isopropanol, n-, t- oder i-Butanol, einem Pentanol oder Hexanol verwendet wird.

11. Verfahren gemäß Anspruch 1 oder 2, **dadurch gekennzeichnet**, daß die Alkoxylierung durch einen primären, sekundären oder tertiären Alkohol mit 4 bis 8 C-Atomen erfolgt.

12. Verfahren gemäß Anspruch 1 oder 2, **dadurch gekennzeichnet**, daß die Alkoxylierung durch n-Butanol, i-Butanol oder sek.-Butanol erfolgt.

13. Verfahren gemäß Anspruch 1 oder 2, **dadurch gekennzeichnet**, daß die Alkoxylierung durch Pentanol, Hexanol, Heptanol oder Octanol erfolgt.

14. 5-Chlor-2-butoxypyridin

## Claims

1. A process for preparing 2,5-dichloropyridine, **characterized in that**
a) 2-chloropyridine or 2-bromopyridine is alkoxylated in the presence of a base, in particular an alkali or alkaline-earth hydroxide, at increased temperature, namely until the reflux temperature, by an alcohol which is liquid at room temperature and which has from 4 to 10 carbon atoms,
b) the alkoxylation product is chlorinated at room temperature in an aqueous suspension in the presence of an alkali or alkaline-earth hydroxide or oxide as an auxiliary base, a catalyst in small quantities and optionally small quantities of emulsifier by the introduction of chlorine gas,
c) the resulting isomer mixture is treated with a Vilsmeyer-Haack reagent,
d) the 2,5- and 2,3-isomer mixture obtained in this way is subjected to a water-vapour distillation, and
e) the crystalline product is re-crystallized from an alcohol/water mixture.

2. A process for preparing pure 2,5-dichloropyridine according to Claim 1, **characterized in that** the resulting distilled isomer mixture is absorbed in an aromatic solvent and 2-alkoxy-5-Cl-pyridine is separated from the 3-isomer by the introduction of gaseous hydrogen halide and the precipitation of the resulting hydrochloride, the 5-isomer is treated with a Vilsmeyer-Haack reagent, the crude 2,5-dichloropyridine obtained is subjected to a water-vapour distillation, and the crystalline product is re-crystallised from an alcohol/water mixture.

3. A process according to Claim 2, **characterized in that** the 2-alkoxy-3-Cl-pyridine separated from the 2-alkoxy-5-Cl-pyridine and remaining in solution by the introduction of gaseous hydrogen halide
a) is released from the solvent by distillation,
b) is treated with a Vilsmeyer-Haack reagent, [and]
c) is subjected to a water-vapour distillation, and
d) the oil enriched with 2,3-dichloropyridine is re-processed in a known manner.

4. A process according to Claim 1 or 2, **characterized in that** 2-chloropyridine or 2-bromopyridine is alkoxylated in the presence of a base, in particular an alkali or alkaline-earth hydroxide, at a temperature of < 110°C.

5. A process according to Claim 1 or 2, **characterized in that** Li₂O, MgO, CaO, BaO, NaOH, KOH, LiOH, Mg(OH)₂, Ca(OH)₂, Ba(OH)₂, Na₂CO₃, K₂CO₃, Li₂CO₃, MgCO₃, CaCO₃, BaCO₃ or an alkali or alkaline-earth acetate is used as the base or the auxiliary base.

6. A process according to Claim 1 or 2, **characterized in that** iodine or bromine in a quantity of less than 1%, relative to the molar quantity of the alkoxypyridine introduced, is used as the catalyst.

7. A process according to Claim 1 or 2, **characterized in that** an emulsifier remaining reactive in the presence of chlorine is used in a quantity of less than 1%, relative to the quantity of the alkoxypyridine introduced.

8. A process according to Claim 1 or 2, **characterized in that** an alkyl sulphonate with from 10 to 18 carbon atoms in a quantity of less than 1%, relative to the quantity of the alkoxypyridine introduced, is used as the emulsifier.

9. A process according to Claim 1 or 2, **characterized in that** the chlorinating ether cleavage is performed, in that one part of 2-alkoxy-chloropyridine is mixed so as to react with from 2·5 to 3·5 parts of POCl₃ in from 2 to 4 parts of DMF, relative to the molar quantity.

10. A process according to Claim 1 or 2, **characterized in that** a mixture of water and an alcohol from the group of sec. or tert. isopropanol, n-, t- or i-butanol, a pentanol or hexanol is used for re-crystallization.

11. A process according to Claim 1 or 2, **characterized in that** the alkoxylation is performed by a primary, secondary or tertiary alcohol with from 4 to 8 carbon atoms.

12. A process according to Claim 1 or 2, **characterized in that** the alkoxylation is performed by n-butanol, i-butanol or sec. butanol.

13. A process according to Claim 1 or 2, **characterized in that** the alkoxylation is performed by pentanol, hexanol, heptanol or octanol.

14. 5-chloro-2-butoxypyridine

## Revendications

1. Procédé pour la préparation de 2,5-dichloropyridine, caractérisé en ce que
a) de la 2-chloropyridine ou de la 2-bromopyridine est alcoxylée par un alcool ayant 4 à 10 atomes de C, liquide à la température ambiante, en présence d'une base, notamment d'un hydroxyde alcalin ou alcalino-terreux à température accrue et ce jusqu'à la température de reflux,
b) le produit d'alcoxylation est chloré à température ambiante en suspension aqueuse par introduction de chlore gazeux en présence d'un oxyde ou d'un hydroxyde alcalin ou alcalino-terreux en tant que base auxiliaire, d'un catalyseur en de très faibles quantités et éventuellement en présence de très faibles quantités d'un émulsifiant,
c) le mélange d'isomères obtenu est traité à l'aide d'un réactif de Vielsmeyer-Haack,
d) le mélange d'isomères 2,5 et 2,3 ainsi obtenu est soumis à une distillation à la vapeur, et que
e) le produit cristallin est recristallisé dans un mélange alcool/eau.

2. Procédé pour la préparation de 2,5-dichloropyridine pure selon la revendication 1, caractérisé en ce que le mélange d'isomères produit et distillé est repris dans un solvant aromatique et que la 2-alcoxy-5-Cl-pyridine est séparée de l'isomère 3 par introduction d'un hydracide halogéné gazeux et précipitation du chlorhydrate formé, que l'isomère 5 est traité par un réactif de Vielsmeyer-Haack, que la 2-5-dichloropyridine brute obtenue est soumise à une distillation à la vapeur et que le produit cristallin est recristallisé dans un mélange alcool/eau.

3. Procédé selon la revendication 2, caractérisé en ce que la 2-alcoxy-3-Cl-pyridine demeurant en solution, séparée de la 2-alcoxy-5-Cl-pyridine par introduction d'un hydracide halogéné gazeux,
a) est débarrassée du solvant par distillation,
b) est traitée par un réactif de Vielsmeyer-Haack,
c) est soumise à une distillation à la vapeur, et
d) l'huile enrichie en 2,3-dichloropyridine est traitée selon la manière connue.

4. Procédé selon la revendication 1 ou 2, caractérisé en ce que la 2-chloropyridine ou la 2-bromopyridine est alcoxylée en présence d'une base, notamment d'un hydroxyde alcalin ou alcalino-terreux à une température < 100°C.

5. Procédé selon la revendication 1 ou 2, caractérisé en ce qu'en tant que base ou base auxiliaire, on utilise Li₂O, MgO, CaO, BaO, NaOH, KOH, LiOH, Mg(OH)₂, Ca(OH)₂, Ba(OH)₂, Na₂CO₃, K₂CO₃, Li₂CO₃, MgCO₃, CaCO₃, BaCO₃ ou un acétate alcalin ou alcalino-terreux.

6. Procédé selon la revendication 1 ou 2, caractérisé en ce qu'en tant que catalyseur, on utilise de l'iode ou du brome en une quantité inférieure à 1%, rapportée à la quantité molaire de l'alcoxypyridine mise en oeuvre.

7. Procédé selon la revendication 1 ou 2, caractérisé en ce que l'on utilise un émulsifiant demeurant actif en présence de chlore en une quantité inférieure à 1%, rapportée à la quantité de l'alcoxypyridine mise en oeuvre.

8. Procédé selon la revendication 1 ou 2, caractérisé en ce qu'en tant qu'émulsifiant, on utilise un alkylsulfonate ayant 10 a 18 atomes de C en une quantité inférieure à 1%, rapportée à la quantité de l'alcoxypyridine mise en oeuvre.

9. Procédé selon la revendication 1 ou 2, caractérisé en ce que l'on procède à la scission chlorante de l'éther en mélangeant, en vue de la réaction, une partie de 2-alcoxychloropyridine avec 2,5 à 3,5 parties de POCl₃ dans 2 à 4 parties de DMF, rapportées à la quantité molaire.

10. Procédé selon la revendication 1 ou 2, caractérisé en ce que, pour la recristallisation, on utilise un mélange d'eau et d'un alcool du groupe de l'isopropanol sec. ou tert., de n-, t- ou i-butanol, d'un pentanol ou d'un hexanol.

11. Procédé selon la revendication 1 ou 2, caractérisé en ce que l'alcoxylation s'effectue avec un alcool primaire, secondaire ou tertiaire ayant 4 à 8 atomes de C.

12. Procédé selon la revendication 1 ou 2, caractérisé en ce que l'alcoxylation s'effectue avec du n-butanol, de l'i-butanol ou du butanol sec.

13. Procédé selon la revendication 1 ou 2, caractérisé en ce que l'alcoxylation s'effectue avec du pentanol, de l'hexanol, de l'heptanol ou de l'octanol.

14. 5-chloro-2-butoxypyridine.
